# EUROPEAN PATENT SPECIFICATION

(11) **EP 0 714 431 B1**
(45) Date of publication and mention of the grant of the patent: **08.01.2003**
(21) Application number: 95900353.4
(22) Date of filing: 17.08.1994
(51) Int. Cl.: C10M 101/04, C10M 109/02, C11C 3/00, C07C 69/74

(54) **IMPROVED TELOMERIZED OIL PRODUCT**
VERBESSERTES, TELOMERISIERTES ÖLPRODUKT
HUILE TELOMERISEE ET AMELIOREE

(30) Priority: 18.08.1993 US 108477
(43) Date of publication of application: 05.06.1996
(73) Proprietor: INTERNATIONAL LUBRICANTS, INC., Seattle Washington 98124 (US)
(72) Inventor: SHANAHAN, Alka, Seattle, WA 98166 (US); LANDIS, Philip, S., Alexandria, VA 22312 (US)
(74) Representative: McCarthy, Denis Alexis
(86) International application number: PCT/US94/09274
(87) International publication number: WO 95/005728

(56) References cited:
- GB-A- 2 134 923
- US-A- 5 229 023

## Description

### Technical Field of the Invention

The present invention provides lubricant compositions and lubricant ingredients comprising an improved telomerized oil product. More specifically, the present invention provides a telomerized oil product that is telomerized from a polyunsaturated triglyceride plant-derived oil that predominantly comprises fatty acids having conjugated double bonds. The present invention further provides derivative lubricant products and ingredients made from a polyunsaturated triglyceride plant-derived oil that predominantly comprises fatty acids having conjugated double bonds.

### Background of the Invention

The field of lubricant additives has seen a wide variety of materials used to reduce friction and wear between moving parts. Lubricants are composed principally of a base stock and a lubricant additive. The lubricant additive provides the antifriction and antiwear characteristics to the lubricant. The base stock imparts improved viscosity and thermal oxidative stability, which can be improved by the addition of various additives. One significant advance in the field was the invention of a material called a "telomer". The telomer invention is described in WO92/07051 and in United States Patent 5,229,023.

Briefly, a telomer is a polymerized triglyceride oil, principally derived from a seed oil, that has thermal oxidative stability and viscosity improvement characteristics that makes the telomer an essential component of a large variety of lubricant compositions. The process to synthesize telomers begins with a triglyceride and heats the oil in a non-oxidizing atmosphere with a trace water catalyst to lower the iodine number such that no more than 4% of the fatty acid chains of the telomerized vegetable oil are polyunsaturated. The triglyceride vegetable oils are characterized as having from 10% to 75% polyunsaturated fatty acid chains of from 16 to 26 carbon atoms in length.

The present invention was made in an effort to improve the telomer product by improving viscosity and colour characteristics of the telomer product.

### Summary of the Invention

The present invention provides an improved telomer product and an improved process for synthesizing the telomer product. More specifically, the present invention provides a triglyceride oil wherein at least 25% of the fatty acids comprise at least two conjugated double bonds that is formed into a telomer by heating the oil for from 3 hours to 10 hours at a temperature of from 150 °C to 400 °C. Preferably, at least 25% of the fatty acids comprise at least three conjugated double bonds.

The improved telomer product is useful as an antiwear agent and as a thickening agent in, lubricant compositions.

The present invention provides an improved telomerized oil produced by a process comprising heating from 20% to 70% of a conjugated triglyceride oil, wherein the conjugated triglyceride oil has at least 25% of fatty acids polyunsaturated with at least two conjugated double bonds and from 30% to 80% of a vegetable triglyceride oil, wherein the vegetable triglyceride oil has from 10% to 75% of its fatty acids being polyunsaturated and having from 6 to 26 carbon atom chain length (unbranched), in a non- oxidizing atmosphere at from 150 °C to 400 °C for from 3 hours to 10 hours to lower the total number of polyunsaturated fatty acids in the conjugated triglyceride oil and vegetable triglyceride oil to less than 10% through the formation of aliphatic rings. Preferably the conjugated triglyceride oil comprises at least three conjugated double bonds in at least 50% of its fatty acids. Preferably, the total number of polyunsaturated fatty acids in the improved telomerized oil is less than 4%

The present invention further provides a process for synthesizing an improved telomerized oil, comprising heating from 20% to 70% of a conjugated triglyceride oil, wherein the conjugated triglyceride oil has at least 25% of fatty acids polyunsaturated with at least two conjugated double bonds and from 30% to 80% of a vegetable triglyceride oil, wherein the triglyceride vegetable oil has from 10% to 75% of its fatty acids being polyunsaturated and having from 16 to 26 carbon atom chain length (unbranched), in a non-oxidizing atmosphere at from 150 °C to 400 °C for from 3 hours to 10 hours to lower the total number of polyunsaturated fatty acids in the conjugated triglyceride oil and vegetable triglyceride oil to less 4% through the formation of aliphatic rings. Preferably the conjugated triglyceride oil comprises at least three conjugated double bonds in at least 25% of its fatty acids.

### Detailed Description of the Invention

The present invention represents a surprising improvement over the telomer oil art and provides an improved telomerized oil using a process that requires a lower processing temperature and a faster reaction time than the original telomer invention described in United States Patent 5, 229,023. Moreover, the present invention provides an improved telomerized oil having improved viscosity, lighter color and a lower acid number than the telomerized oil described in United States Patent 5, 229,023. The telomerized oil described in United States Patent 5, 229,023 is characterized as the product of heating a vegetable triglyceride oil for at least 5 hours at a temperature of from 200 °C to 400 °C to lower the number of polyunsaturated fatty acids to less than 4% of the total number of fatty acids through formation of aliphatic rings, wherein the vegetable triglyceride oil has from 10% to 75% of its fatty acids being polyunsaturated and having from 16 to 26 carbon atoms chain lengths. Several appropriate vegetable triglyceride oils were illustrated, such as those vegetable triglyceride oils selected from the group consisting of rapeseed oil, crambe oil, meadowfoam oil, soya bean oil, peanut oil, corn oil, safflower oil, sunflower seed oil, cottonseed oil, olive oil, coconut oil, palm oil, linseed oil, and combinations thereof.

The present invention found a surprising improvement in the properties and processing characteristics of the resulting telomerized oil by mixing from 20% to 70% of a conjugated triglyceride oil into the vegetable triglyceride oil for heating to form the telomerized oil. The conjugated triglyceride oil has at least 25% of fatty acids having at least two conjugated double bonds. Preferably, the conjugated triglyceride oil has at least three conjugated double bonds. By a conjugated double bond, this terms refers to a fatty acid having an alternating single bond and double bond along the hydrocarbon chain. The resulting improved telomerized oil has increased viscosity, increased solubility in a variety of lubricant base oils, is lighter in color and has a lower acid number (a measure of the amount of free fatty acids present). The lighter color will provide greater consumer acceptance, as darker colors denote a more oxidized product. Also, the acid number is an indication of oxidation breakdown, so it is desirable to have a product start with a lower acid number, especially when the material is to be used in a lubricant operating in a high temperature environment, such as an engine oil. Moreover, the resulting improved telomerized oil can be processed at a lower temperature and will telomerize faster resulting in a shorter reaction time. This provides significant energy savings and reduced processing and operating costs operating at a lower temperature for a shorter time.

The essential characteristic of the conjugated triglyceride oil is the presence of conjugated double bonds in at least 25% of the fatty acids in the triglyceride oil. Examples of appropriate conjugated triglyceride oils include, for example, tung oil, oitcica oil, seed fats of *Rosaceae*, *Euphorbiaceae*, and *Cucurbitaceae* families, fish oils enriched in ω-3 fatty acids, and combinations thereof. Tung oil was used as an example of a conjugated triglyceride oil. Tung oil is made from kernels of the fruit of the tung tree, which has been grown in China for centuries and more recently (since 1925) grown in southeastern United States. The tung kernels have about a 17.5% oil content. Tung oil generally has a saponification number of 189-195, an iodine number of 160-175, an R.I. at 25 °C of 1.516-1.520 and unsaponified matter below 1%. The fatty acid profile of tung oil is shown in Table 1 below.

**Table 1**

| **Fatty Acid** | **Percent (%)** |
|---|---|
| oleic acid | 4-9 |
| linoleic acid | 8-10 |
| saturated | 2-6 |
| α-eleostearic acid | 77-86 |
| linolenic acid | trace |

α-Eleostearic acid is a conjugated fatty acid, such as 9,11,13-octadecatrienoic acid, and linolenic acid is predominantly (not conjugated) 9,12,15-octadecatrienoic acid. The cold water fish oils (and cod liver oil) contain high levels of the polyunsaturated fatty acids eicosapentaenoic acid and docosahexaenoic acid.

Alternatively, conjugated fatty acids in a triglyceride format can be obtained in a two-step synthetic process to form triglyceride oils with a conjugated diene structure. Specifically, monounsaturated plant oils (*e.g*., HEAR or high erucic acid rape seed oil, corn oil, crambe oil, meadowfoam oil, linseed oil, or other plant seed oils having predominantly monunsaturated fatty acids such as oleic acid) are halogenated (preferably chlorinated) and dehydrohalogenated (preferably with a base such as NaOH, KOH or Ca(OH)₂) to yield a mixture of monounsaturated fatty acids and conjugated diene fatty acids in a triglyceride structure. This then creates an ideal mixture for telomerization using the inventive procedure.

Specifically, a monounsaturated plant oil, defined as a triglyceride oil having at least 60% monoene fatty acids of C₁₄₋₂₂ (for example, rapeseed, corn, crambe, meadowfoam, linseed oils, etc.), is halogenated by adding halogen (for example, chlorine, bromine, iodine or combinations thereof, preferably chlorine) to a solution of the monounsaturated plant oil in from 0-60% by volume of halogenated solvent (defined as a halogenated solvent comprising no more than three carbon atoms, preferably wherein the halogen is chlorine and there are one or two carbon atoms (*e.g.*, chloroform, carbon tetrachloride, methylenechloride, etc.)). The specific temperature of the halogenation reaction should be above the solidification point of the oil or oil in solvent mixture and not to exceed 50 °C, as temperature above 50 °C tend to provide substitution reactions rather than the required addition of halogen atoms across a double bond. The halogenation reaction (addition reaction) is run until at least 30% (by weight) of the halogen used is taken up. The addition reaction is stopped by treating the oil mixture with a base and stirred for 2-4 hours. Preferred bases are NaOH, KOH, Ca(OH)₂ and combinations thereof. The product formed is a mixture of triglyceride oils containing monoene and conjugated diene structures plus salt (*e*.*g*., NaCI if chlorine was the halogen and NaOH the base) and any remaining solvent. The oil product is filtered to remove the salt and stripped to remove any remaining solvent. This oil product can then be used for the improved telomerization process described herein as a substitute for a natural conjugated triglyceride oil product. The advantage of this alternative "synthetic" process over obtaining a natural source of conjugated triglyceride oil is cost and availability.

There is a need to improve the viscosity of lubricant products such that the viscosity of the lubricant product does not break down when operating at high temperatures (such as an engine oil). Such ingredients are called "VI" improvers for viscosity index. There is a problem with VI improvers that are not soluble in a variety of base oils, such as various mineral oil bases and seed oil base oils. The improved telomerized oil has greater solubility in a variety of base oils that a telomerized vegetable oil, including solubility in a seed oil (HEAR oil, a rapeseed oil), a napthenic oil, a paraffinic oil and a eicosyl erucate linear liquid wax ester (EG-20 oil).

A group of telomerized vegetable oils and improved telomerized oils were made. The raw materials, reaction time, and reaction temperatures are listed in Table 2 below:

**Table 2**

| **Product** | **Raw Material (wt. %)** | | | | |
|---|---|---|---|---|---|
| | Tung oil | Linseed oil | HEAR | Temp °C | Time (hr) |
| A | 0 | 50 | 50 | 320 | 6 |
| B | 0 | 50 | 50 | 293 | 6 |
| C | 50 | 0 | 50 | 293 | 6 |
| D | 40 | 0 | 60 | 293 | 6 |
| E | 50 | 0 | 50 | 293 | 6 |
| F | 30 | 35 | 35 | 293 | 6 |
| G | 30 | 35 | 35 | 303 | 6 |
| H | 40 | 0 | 60 | 293 | 6 |
| I | 0 | 50 | 50 | 320 | 24 |
| J | 50 | 0 | 50 | 225 | 4.5 |
| K | 50 | 0 | 50 | 300 | 0.2 |
| L | 50 | 0 | 50 | 300¹/225 | 0.5 |
| M | 50 | 0 | 50 | 300¹/225 | 2.5 |
| N | 50 | 0 | 50 | 300¹/225 | 3.0 |
| O | 50 | 0 | 50 | 300¹/225 | 4.0 |
| P | 50 | 0 | 50 | 225¹/190 | 6.5 |

| | | | | | |
|---|---|---|---|---|---|
| where¹ refers to an initiation temperature for the reaction. Telomerized oils A and B are vegetable telomerized oils described in United States Patent 5,229,023. Telomerized oils C-H are improved telomerized oils. The results of KV (kinematic viscosity), acid number (mg KOH/g) and solubility at 5% in a variety of base oils show the improved properties of the improved telomerized oil. Table 3 below illustrate these data. | | | | | |

**Table 3**

| | | | | **Solubility @5%** | | | |
|---|---|---|---|---|---|---|---|
| Oil | KV@40 °C(mm²s⁻¹) | KV@40 °C (sus) | Acid No. | EG-20 | Naphthenic | Parrafrmic | HEAR |
| **A** | 571 | 2636 | 23.99 | clear | clear | clear | clear |
| **B** | 140 | 646 | 7.65 | clear | clear | clear | clear |
| **C** | 8,945 | 41,328 | 5.79 | clear | clear | clear | clear |
| **D** | 1,382 | 6387 | 4.99 | clear | clear | clear | clear |
| **E** | 12,286 | 56,760 | 5.21 | clear | clear | clear | clear |
| **F** | 2,885 | 13,330 | 4.68 | clear | clear | clear | clear |
| **G** | 3,817 | 17,634 | 10.1 | clear | clear | clear | clear |
| **H** | 1,482 | 6848 | 4.85 | clear | clear | clear | clear |
| **I** | 2,165 | 10,000 | 49.3 | clear | clear | hazy | clear |

For the solubility measurements, napthenic oil was a Shell MVI oil, paraffinic oil was a Mohawk 150, and EG-20 was a linear liquid was ester, and the HEAR oil used was a triglyceride rapeseed vegetable oil. Moreover, products J, K, L, M, N, O, and P had kinematic viscosity (40°C) mm²s⁻¹ measurements of 2,165, 433, 945,2,081,2,707,4,267 and 2,642 mm²s⁻¹ respectively (40 °C sus measurements of 10,000, 2,000, 4,365, 9,615, 12,508, 19,713, and 12,205, respectively). All of products J-P were completely soluble in EG-20, Naphthenic, Paraffinic and HEAR oils at 5% (by weight), and yielded an acid number of <3.

### EXAMPLE 1

This example illustrates an evaluation of various properties of an improved telomer oil. The improved telomer oil was made from tung oil (50% ) and HEAR (high erucic acid rapeseed oil) (50% ), and heated to about 293 °C for a six hour reaction. The kinematic viscosity was 8,922 mm²s⁻¹ (8922 cSt) and 8,945 mm²s⁻¹ (41328 saybolt universal seconds, sus) at 40 °C, and 3,806 mm²s⁻¹ (3806 cSt) and 3,816 mm²s⁻¹ (17630 sus) at 100 °C, indicating a substantial improvement in viscosity over a telomer oil made with only HEAR and without a conjugated polyunsaturated triglyceride oil. The acid value was 5.79 mg KOH/gm, and the color was golden yellow.

The solubility characteristics of the improved telomer oil are soluble (at 5% ) in HEAR oil, EG-20 (a rapeseed oil-derived linear liquid wax ester), a naphthenic-based MVI (Shell), and a paraffinic-based MVI (Mohawk 150). As a comparison, a telomerized oil made without a conjugated polyunsaturated triglyceride oil and with rapeseed oil and a source of triglyceride linoleic acid was soluble at 5% in HEAR oil, EG-20, and the Naphthenic-based MVI, but not the paraffinic-based MVI.

The improved telomer oil (called T -41000), a telomerized vegetable oil (product A in Table 2), and a commercially available VI improver (Acryloid 1019, a polymethacrylate based and shear stable VI improver, kinematic viscosity of 800 ± 150 mm²s⁻¹ (800±150 Cst) at 100 °C) were compared for thickening effect at 2% in a typical MVI oil (Shell). Both products significantly improved the kinematic viscosity of the final blend. The comparison are present in Table 4 below:

**Table 4**

| **Kinematic** **Viscosity** | **MVI** | **MVI + 2% T-41000** | **MVI + 4%** **Prod. A** | **MVI + 2%** **Acryloid** |
|---|---|---|---|---|
| mm2s-1@40oC | 22.36 | 25.81 | 24.52 | 24.77 |
| mm²s⁻¹@100°C | 4.06 | 4.58 | 4.43 | 4.53 |
| cSt@40°C | 22.36 | 25.81 | 24.52 | 24.77 |
| cSt@100°C | 4.06 | 4.58 | 4.43 | 4.53 |
| Viscosity Index | 61 | 85 | 83 | 92 |

### EXAMPLE 2

This example illustrates a comparison of antiwear characteristics of a telomerized vegetable oil and an inventive improved telomer oil. The telomerized vegetable oil and improved telomer oil are described in Example I. Antiwear properties were detennined using a Falex Pin and Vee Block method (ASTM D-2670). One percent of each telorner oil was mixed into a base fluid, consisting of Mohawk 150 oil (30%), bright stock (69.75%) and Mobil E.P additive (0.25%) by weight. The results (presented in Table 5 below) show improved antiwear properties of the improved telomer oil over the vegetable oil telornerized oil.

**Table 5**

| Sample | Teeth Wear | % Reduction |
|---|---|---|
| Basic fluid control | 71 | - |
| 1% Vegetable Oil Telomer | 30 | 57.7% |
| 1% Improved Telomer Oil | 8 | 89 % |

Therefore, the improved telomer exhibits improved antiwear characteristics over the original vegetable oil telomer, yet still retains its viscosity improvement and thermal oxidative stability characteristics.

### EXAMPLE 3

This example illustrates a group of reactions wherein the reaction mixture was stirred at higher rates in an effort to better distribute the heat of the reaction and thereby lower the reaction temperatures and times. In each reaction, an inert atmosphere, composed of a blanket of nitrogen, was bubbled into the oil. Each reaction oil was a mixture of 750g of HEAR (high erucic acid rapeseed oil) and 740g of tung oil. The reactions were done at 224 to 225 °C. All final products were soluble in HEAR and EG-20.

**Table 6**

| **Time of Reaction (hrs)** | **Viscosity** @ **40°C** | | **NN (acid no.)** |
|---|---|---|---|
| | mm²s⁻¹ | (sus) | |
| 1 | 144 | 667 | |
| 2 | 239 | 1103 | |
| 3 | 382 | 1765 | |
| 4 | 700 | 3235 | |
| 5 | 2,035 | 9400 | 1.8 |

The above reactions were run again, except this time nitrogen was blanketed over the oil without being bubbled through the oil. Each reaction oil was a mixture of 100g HEAR and 990 g of tung oil and the temperature was 215-222 °C. All final products were soluble in HEAR and EG-20.

**Table 7**

| **Time of Reaction (hrs)** | **Viscosity @ 40°C mm**^{**2**}**s**^{**-1**} | **Viscosity @ 40°C, sus** |
|---|---|---|
| 1 | 104 | 480 |
| 2 | 163 | 753 |
| 4 | 442 | 2040 |
| 5 | 923 | 4264 |
| 5.5 | 1,618 | 7477 |
| 5.75 | 2,498 | 11,540 |
| 6 | 5,996 | 27,700 |

## Claims

1. A telomerized oil producible by a process comprising heating from 20% to 70% (by weight) of a conjugated triglyceride oil, wherein the conjugated triglyceride oil has at least 25% of fatty acids polyunsaturated with at least two conjugated double bonds and from 30% to 80% (by weight) of a vegetable triglyceride oil, wherein the vegetable triglyceride oil has from 10% to 75% of its fatty acids being polyunsaturated and having from 16 to 26 carbon atom chain length (unbranched), in a non-oxidizing atmosphere for from 150°C to 400°C for from 3 hours to 10 hours to lower the total number of polyunsaturated fatty acids in the conjugated triglyceride oil and the vegetable triglyceride oil to less than 10% through the formation of aliphatic rings.

2. The telomerized oil of claim 1, wherein the conjugated triglyceride oil is obtained from tung oil, fish oils enriched in ω-3 fatty acids, cod liver oil, or it is synthesized from a vegetable triglyceride oil, and or it is a combination thereof.

3. The telomerized oil of claims 1 or 2, wherein the vegetable triglyceride oil is obtained from rapeseed oil, crambe oil, meadowfoam oil, soya bean oil, peanut oil, corn oil, safflower oil, sunflower seed oil, cottonseed oil, olive oil, coconut oil, palm oil, linseed oil, and combinations thereof.

4. A process for synthesizing an telomerized oil, comprising heating from 20% to 70% (by weight) of a conjugated triglyceride oil, wherein the conjugated triglyceride oil has at least 25% of fatty acids polyunsaturated with at least two conjugated double bonds and from 30% to 80% (by weight) of a vegetable triglyceride oil, wherein the vegetable triglyceride oil has from 10% to 75% of its fatty acids being polyunsaturated and having from 16 to 26 carbon atom chain length, in non-oxidizing atmosphere at from 150°C to 400°C for from 3 hours to 10 hours to lower the amount of polyunsaturation in the conjugated triglyceride oil and vegetable triglyceride oil to less than 10% through the formation of aliphatic rings.

5. The process or claim 4, wherein the vegetable triglyceride oil is obtained from rapeseed oil, crambe oil, meadowfoam oil, soya bean oil, peanut oil, corn oil, safflower oil, sunflower seed oil, cottonseed oil, olive oil, coconut oil, palm oil, linseed oil, and combinations thereof.

6. The process of claim 4 wherein the amount of polyunsaturation in the conjugated triglyceride oil and vegetable triglyceride oil is lowered to less than 4% through the formation of aliphatic rings.

7. A process for synthesizing a telomerized oil from a non-conjugated vegetable triglyceride oil source material, comprising (1) halogenating the non-conjugated vegetable triglyceride oil at 0-50°C to form a halogenated oil, (2) treating the halogenated oil with base to form a salt with from 30-60% conjugated double bond fatty acids in the oil with at least 25% of the fatty acids polyunsaturated with at least two conjugated double bonds, and (3) heating, in a non-oxidizing atmosphere at from 150°C to 400°C for from 3 hours to 10 hours to lower the amount of polyunsaturation in the oil to less than 10% through the formation of aliphatic rings.

8. The process of claim 7 wherein the non-conjugated vegetable triglyceride oil is selected from the group consisting of HEAR or high erucic acid rape seed oil, corn oil, crambe oil, meadowfarm oil, linseed oil, or other plant seed oils having predominantly monounsaturated fatty acids.

9. The process of claim 7 wherein the halogen is chlorine and the base is selected from the group consisting of NaOH, KOH and Ca(OH)₂.

10. The process of claim 7 wherein the non-conjugated vegetable triglyceride oil is diluted in up to 60% of a solvent, wherein the solvent is halogenated solvent, comprising no more than three carbon atoms.

## Patentansprüche

1. Telomerisiertes Öl, herstellbar nach einem Verfahren, umfassend:
ein Erwärmen von 20 Gew.-% bis 70 Gew.-% von einem konjugierten Trigglyceridöl, wobei mindestens 25% der Fettsäuren des konjugierten Trigglyceridöl mehrfach ungesättigt sind mit mindestens zwei konjugierten Doppelbindungen, und von 30 Gew.-% bis 80 Gew.-% von einem pflanzlichen Triglyceridöl, wobei 10% bis 75% der Fettsäuren des pflanzlichen Triglyceridöls mehrfach ungesättigt sind und eine Kettenlänge (unverzweigt) von 16 bis 26 Kohlenstoffatomen aufweisen, in einer nicht-oxidierenden Atmosphäre bei von 150°C bis 400°C über einen Zeitraum von 3 Stunden bis 10 Stunden, um die Gesamtzahl der mehrfach ungesättigten Fettsäuren in dem konjugierten Trigglyceridöl und dem pflanzlichen Triglyceridöl auf weniger als 10% durch die Bildung von aliphatischen Ringen zu erniedrigen.

2. Das telomerisierte Öl nach Anspruch 1, wobei das konjugierte Öl erhalten wird aus Tungöl, Fischölen angereichert an ω-3-Fettsäuren, Dorschleberöl, oder es ist synthetisiert aus einem pflanzlichen Triglyceridöl, und/oder es ist eine Kombination davon.

3. Das telomerisierte Öl nach Anspruch 1 oder 2, wobei das pflanzliche Triglyceridöl erhalten wird aus Rapsöl, Öl des See- oder Strandkohls (*Crambe maritima;* Engl.: crambe), Öl von *Limnanthes douglasii* (Engl.: meadowfoam), Soyabohnenöl, Erdnussöl, Maisöl, Färberdistelöl, Sonnenblumenkernöl, Baumwollkernöl, Olivenöl, Kokosnussöl, Palmöl, Leinöl und Kombinationen davon.

4. Verfahren zur Synthese oder Herstellung eines telomerisierten Öls, umfassend:
ein Erwärmen von 20 Gew.-% bis 70 Gew.-% von einem konjugierten Trigglyceridöl, wobei mindestens 25% der Fettsäuren des konjugierten Trigglyceridöl mehrfach ungesättigt sind mit mindestens zwei konjugierten Doppelbindungen, und von 30 Gew.-% bis 80 Gew.-% pflanzlichem Triglyceridöl, wobei 10% bis 75% der Fettsäuren des pflanzlichen Triglyceridöls mehrfach ungesättigt sind und eine Kettenlänge von 16 bis 26 Kohlenstoffatomen aufweisen, in einer nicht-oxidierenden Atmosphäre bei von 150°C bis 400°C über einen Zeitraum von 3 Stunden bis 10 Stunden, um die Menge an mehrfach ungesättigten Bindungen in dem konjugierten Trigglyceridöl und dem pflanzlichen Triglyceridöl auf weniger als 10% durch die Bildung von aliphatischen Ringen zu erniedrigen.

5. Das Verfahren nach Anspruch 4, wobei das pflanzliche Triglyceridöl erhalten wird aus Rapsöl, Öl des See- oder Strandkohls (*Crambe maritima*; Engl.: crambe), Öl von *Limnanthes douglasii* (Engl.: meadowfoam), Soyabohnenöl, Erdnussöl, Maisöl, Färberdistelöl, Sonnenblumenkernöl, Baumwollkernöl, Olivenöl, Kokosnussöl, Palmöl, Leinöl und Kombinationen davon.

6. Das Verfahren nach Anspruch 4, wobei die Menge an mehrfach ungesättigten Bindungen in dem konjugierten Trigglyceridöl und dem pflanzlichen Triglyceridöl auf weniger als 4% erniedrigt wird durch die Bildung von aliphatischen Ringen.

7. Verfahren zur Synthese oder Herstellung eines telomerisierten Öls aus einem Ausgangsmaterial von nicht-konjugiertem pflanzlichen Triglyceridöl, umfassend: (1) ein Halogenieren des nicht-konjugiertem pflanzlichen Triglyceridöls bei 0-50°C, um ein halogeniertes Öl zu bilden, (2) ein Behandeln des halogenierten Öls mit einer Base, um ein Salz zu bilden mit von 30-60% Fettsäuren mit konjugierten Doppelbindungen in dem Öl, wobei mindestens 25% der Fettsäuren mit mindestens zwei konjugierten Doppelbindungen mehrfach ungesättigt sind, und (3) einem Erwärmen in einer nicht-oxidierenden Atmosphäre bei von 150°C bis 400°C über einen Zeitraum von 3 Stunden bis 10 Stunden, um die Menge an mehrfach ungesättigten Bindungen in dem Öl auf weniger als 10% durch die Bildung von aliphatischen Ringen zu erniedrigen.

8. Das Verfahren nach Anspruch 7, wobei das nicht-konjugierte pflanzliche Öl ausgewählt ist aus der Gruppe, bestehend aus HEAR oder Rapsöl mit großem Anteil an Erukasäure (Engl.: high erucic acid rape seed oil), Maisöl, Öl des See- oder Strandkohls (*Crambe maritima;* Engl.: crambe), Öl von *Limnanthes douglasii* (Engl.: meadowfoam), Leinöl oder anderen Pflanzensamen- oder Pflanzenkernölen, die überwiegend einfach ungesättigte Fettsäuren aufweisen.

9. Das Verfahren nach Anspruch 7, wobei das Halogen Chlor ist und die Base ausgewählt ist aus der Gruppe, bestehend aus NaOH, KOH und Ca(OH)₂.

10. Das Verfahren nach Anspruch 7, wobei das nicht-konjugierte pflanzliche Öl verdünnt ist in bis zu 60% von einem Lösungsmittel, wobei das Lösungsmittel ein halogeniertes Lösungsmittel ist, das nicht mehr als drei Kohlenstoffatome umfasst.

## Revendications

1. Huile télomérisée améliorée produite par une méthode comprenant le chauffage d'un mélange formé de 20% à 70% (en poids) d'huile triglycéride conjuguée, dans laquelle 25% au moins des acides gras sont polyinsaturés et possèdent au moins deux liaisons doubles conjuguées, et de 30% à 80% (en poids) d'une huile triglycéride végétale dont 10% à 75% des acides gras sont polyinsaturés et leurs chaînes (non ramifiées) sont formées de 16 à 26 atomes de carbone, dans une atmosphère non oxydante à une température comprise entre 150 °C et 400 °C pendant une durée comprise entre 3 et 10 heures pour ramener le nombre total d'acides gras polyinsaturés dans l'huile triglycéride conjuguée et dans l'huile triglycéride végétale à moins de 10% par formations d'anneaux aliphatiques.

2. Huile télomérisée selon la revendication 1, dans laquelle l'huile triglycéride conjuguée est obtenue à partir d'huile de bois de chine, d'huiles de poisson enrichies en acides gras ω-3, d'huile de foie de morue, ou est synthétisée à partir d'une huile végétale triglycéride et/ou est constituée d'une combinaison de celles-ci.

3. Huile télomérisée selon les revendications 1 ou 2, dans laquelle l'huile triglycéride végétale est obtenue à partir d'huile de colza, d'huile de crambe, d'huile de limnanthe, d'huile de soja, d'huile d'arachide, d'huile de maïs, d'huile de carthame, d'huile de tournesol, d'huile de coton, d'huile d'olive, d'huile de noix de coco, d'huile de palme, d'huile de lin et de leurs combinaisons.

4. Méthode de synthèse d'une huile télomérisée, comprenant le chauffage d'un mélange formé de 20% à 70% (en poids) d'huile triglycéride conjuguée, dans laquelle 25% au moins des acides gras sont polyinsaturés et possèdent au moins deux liaisons doubles conjuguées, et de 30% à 80% (en poids) d'une huile triglycéride végétale dont 10% à 75% des acides gras sont polyinsaturés et leurs chaînes sont formées de 16 à 26 atomes de carbone, dans une atmosphère non oxydante à une température comprise entre 150 °C et 400 °C pendant une durée comprise entre 3 et 10 heures pour ramener la quantité de polyinsaturation dans l'huile triglycéride conjuguée et dans l'huile triglycéride végétale à moins de 10% par formation d'anneaux aliphatiques.

5. Méthode selon la revendication 4, dans laquelle l'huile triglycéride végétale est obtenue à partir d'huile de colza, d'huile de crambe, d'huile de limnanthe, d'huile de soja, d'huile d'arachide, d'huile de maïs, d'huile de carthame, d'huile de tournesol, d'huile de coton, d'huile d'olive, d'huile de noix de coco, d'huile de palme, d'huile de lin et de leurs combinaisons.

6. Méthode selon la revendication 4 dans laquelle la quantité de polyinsaturation dans l'huile triglycéride conjuguée et dans l'huile végétale télomérisée est abaissée à moins de 4% par formation d'anneaux aliphatiques.

7. Méthode de synthèse d'une huile télomérisée à partir d'une matière source d'huile triglycéride végétale non conjuguée, comprenant (1) l'halogénisation de l'huile triglycéride végétale non conjuguée à une température comprise entre 0 et 50 °C pour former une huile halogénée, (2) le traitement de l'huile halogénée par une base pour former un sel avec de 30 à 60% d'acides gras à liaisons doubles conjuguées dans l'huile, 25% au moins des acides gras étant polyinsaturés et possédant au moins deux liaisons doubles conjuguées, et (3) chauffage dans une atmosphère non oxydante à une température comprise entre 150°C et 400 °C pendant une durée comprise entre 3 heures et 10 heures pour ramener la quantité de polyinsaturation dans l'huile à moins de 10% par formations d'anneaux aliphatiques.

8. Méthode de 1a revendication 7 dans laquelle l'huile triglycéride végétale non conjuguée est sélectionnée dans le groupe comprenant l'huile HEAR ou huile de colza à haute teneur en acide érucique, l'huile de crambe, l'huile de limnanthe, l'huile de lin ou d'autres huiles de graines végétales comportant de façon prédominante des acides gras monoinsaturés.

9. Méthode de la revendication 7 dans laquelle l'halogène est le chlore et la base est sélectionnée dans le groupe constitué de NaOH, KOH et Ca(OH)₂.

10. Méthode de la revendication 7 dans laquelle l'huile triglycéride végétale non conjuguée est diluée dans une quantité de solvant représentant jusqu'à 60%, dans laquelle le solvant est halogéné et ne comporte pas plus de trois atomes de carbone.
